# EUROPEAN PATENT APPLICATION

(11) **EP 1 229 044 A1**
(43) Date of publication of application: **07.08.2002**
(21) Application number: 02090043.7
(22) Date of filing: 30.01.2002
(51) Int. Cl.: C07K 14/16, G01N 33/569, C07K 17/12

(54) **Protein having multiple antigen/epitope sequences and being immobilized for the diagnosis of HIV infections**

(30) Priority: 02.02.2001 DE 10106295
(71) Applicant: GAIFAR German American Institute for Applied Biomedical Research GmbH, 14473 Potsdam (DE)
(72) Inventor: Repke, Heinrich, 14089 Berlin (DE); Budde, Eckard, 10997 Berlin (DE); Nicolaus, Stefan, 14473 Potsdam (DE)
(74) Representative: Jungblut, Bernhard Jakob, Dr.

(57) **Abstract**

The invention relates to a protein having multiple antigen/epitope sequences for antibodies, wherein the protein is immobilized at a solid phase by at least one binding site, and the antigen/epitope sequences are spaced by bridge compositions in such a way that after binding of the binding site at the solid phase the antigen/epitope sequences are exposed for a binding of the assigned antibodies from the liquid phase.

## Description

### Field of the invention

The invention relates to a protein having multiple antigen/epitope sequences and being immobilized, to a polynucleotide coding for such a protein, to an expression vector including such polynucleotide, to a cell transformed by means of such an expression vector, and to applications of such a protein for producing an immobilizate and for performing a HIV test.

### Background of the invention

Immobilizates comprising a solid phase and one or more proteins bound to the solid phase are well known by practical experiences. Such immobilizates are used, on one hand, for the detection of substances of a liquid analyzate specifically binding to the protein, and on the other hand for the separation of such substances from a liquid. Therein the protein comprises an epitope being specific for the substance to be detected or to be separated, and this epitope normally binds to the solid phase by a spacer composition per se not binding to the substance. The spacer composition secures, among other effects, that the protein folds in the area of the epitope in a way corresponding to the folding of a native epitope. This, i.e. the desired exposure of the epitope, is the condition for that a binding of the substance is at all possible. In particular, the formation of undesired disulfide bridges is prevented.

In some fields of medicine, it is desirable that various epitopes are immobilized at the same time at the solid phase. This is desirable, for instance, in the case of HIV tests, since only an accumulation of epitopes being specific for antibodies for various HIV1 and HIV2 viruses and sub-types will secure a reliable decision on the basis of the test result about whether or not the tested sample contains HIV antibodies, i.e. whether or not a person is HIV positive.

In principle, the respective proteins, for instance antigens against various HIV antibodies, could be immobilized each for its own at the solid phase. This presents however various problems. The first problem is that a uniform distribution of the proteins, in particular with regard to concentrations, and thus a sufficiently uniform sensitivity for the various antibodies cannot be secured. For various proteins may lead to ousting reactions at the solid phase, with the consequence of substantially disturbing concentration differences at the solid phase, even with equimolar addition. Further, with a higher multitude of different proteins, interactions of the various proteins during immobilization cannot be excluded. All this will disturb, however, if a universal test, for instance for HIV, is to be established. The above will apply in a corresponding manner, of course, to all diseases causing, depending from the classes and sub-types of the infecting organism, immunologically distinguishable antibodies in a body. It is understood that different antibodies against a specific sub-type may also be formed.

In conjunction with HIV, the following is to be noted. The acquired immunodeficiency syndrome (AIDS) is caused by the HIV. The two causative types HIV-1 and HIV-2 detected up to now have a very similar genome structure and infect T cells using a very similar mechanism. They immunologically differ, however, by that antibodies against HIV-1 do not usually present a cross reaction with HIV-2, and vice versa. For HIV-1, further, a series of sub-types are distinguished, with the group M comprising several sub-types (A, B, C, D, E, F and G), and the sub-type O being classified in an own group (O). Even between the antibodies against different sub-types there are immunological differences. It is difficult, therefore, to reliably detect in a single test system all sub-types or antibodies thereagainst. This particularly applies to rapid tests having to be performed without too large experimental expenses. In a rapid test, a reliable result should be obtainable with a single application for instance of a blood sample. In particular in conjunction with HIV, incorrect negative and incorrect positive results must virtually be impossible.

### State of the art

Exemplary for the application of one or more antigens in a mixture against various HIV antibodies is the document US-A-5,830,641.

Rapid tests known up to now usually operate, however, with one single antigen, namely gp41, and are not capable, therefore, to detect all HIV sub-types reliably and at high sensitivity. There is thus a substantial risk of incorrect negative results. Further, they are not temperature-stable over longer periods of time, not suitable for permanent documentation, do not secure a simple and safe disposal, and present an incorrect positive result in case of over-development.

Fusion proteins having more than one epitope for HIV antibodies are *per se* known in the art, for instance from the documents US-A-5,800,822 and US-A-5,310,876. These are proteins used in a solution for laboratory tests. Such laboratory test systems are not suitable, however, for rapid tests, due to the complicated handling and the high risk of incorrect results during execution caused by not properly educated persons.

From the document US-A-4,925,784 is known a fusion protein gag/env which may also be immobilized. Bridge compositions with binding sites for a binding with a solid phase between gag and env cannot be taken out. The fusion protein binds at one side over a bridge to a solid phase.

From the document DE 197 20 914 A1 are known in the art various antigens against antibodies of different sub-types. The antigens may bind to a solid phase. Different antigens are used as a mixture. As far as antigens with multiple epitopes are concerned, they are haptens, i.e. proteins with multiple sequences of a single epitope type.

The document US-A-5,241,047 describes peptides reacting with serums of HIV-positive human guinea pigs. Various epitopes are coupled immediately side by side and may bind by a spacer sequence at the C or N-terminal end to a solid phase. By means of a spacer sequence, spacing of a signal sequence from a carrier material is achieved. One end of the spacer sequence binds to the carrier material, and the other end to an end of the signal sequence. To each spacer sequence is coupled one signal sequence only.

### Technical object of the invention

The invention is based on the object to specify an immobilizable protein, which can be used for a rapid test of the presence of antibodies in a patient's sample and reliably permits in such a rapid test a detection of many to all groups and sub-types of a causative agent or of antibodies thereagainst.

### Principles of the invention

As a solution for this technical object, the invention teaches a protein having multiple antigen/epitope sequences for antibodies, the protein being immobilized at a solid phase by at least one binding site, and the antigen/epitope sequences being spaced by bridge compositions in such a way that after binding of the binding site at the solid phase the antigen/epitope sequences are exposed for a binding of the assigned antibodies from the liquid phase.

The invention is based on a combination of findings. A first finding is that the disadvantages of the application of a mixture of antigens can be avoided by that the antigen/epitopes are combined in a single protein. Then an immobilization can easily take place, without different affinities to the solid phase and/or interactions of different antigens between each other leading to disturbing displacements of the distributions. Then the invention is based on the further finding that it will not be sufficient to just line-up different epitopes and to then bind the product at one end (or both ends) to the solid phase. Rather, it has to be achieved that the epitopes are exposed in a desired manner after the immobilization, i.e. form a secondary and maybe a tertiary structure securing a binding of the assigned antibodies and further permitting a sterical accessibility. Finally, it has been found that this can be achieved by establishing bridge compositions between the epitopes causing on one hand the binding to the solid phase and providing on the other hand the desired exposure. The concept is thus basically the generation of a single protein with several spaced epitopes in turn being separated by interposition of bridge compositions. Further, by adequate selection of the bridge compositions, in particular the structures of the parts of the respective bridge compositions extending on either side of an epitope up to the both-sides binding sites at the solid phase, the epitopes can be folded in a defined way, such that the exposure is reliably secured and also fixed. Finally, it is inherent to the concept of the invention that disturbing interactions of different epitopes of a protein are virtually excluded.

Suitable bridge compositions can easily be calculated, based on the epitope structures or sequences flanking such connections, by using the principles of molecular modeling. Additionally or alternatively, the average man skilled in the art can experimentally test various bridge compositions for suitability, by that the binding capability of antibodies to the epitopes flanking the bridge compositions are tested by conventional methods.

It is understood that a protein according to the invention must have ends. One end may be the end of an epitope being neither directly nor indirectly bound to the solid phase. One end may further be an end of a bridge composition, the end of the bridge composition being the binding site or, referred to the connected epitope, may be placed beyond the binding site. One end may finally also be formed by a tag, in particular an affinity tag.

In principle, there are various possibilities to create a protein according to the invention. For instance, a bridge composition may be formed by insertion of bridge sequences between two antigen/epitope sequences and/or deletion of a partial sequence between two antigen/epitope sequences arranged in a total sequence. The bridge composition may also be formed by fusion of a bridge sequence with two antigen/epitope sequences.

Bridge compositions may be various molecules. Basically, any organic substances, typically chain type connections, may be used. Examples are oligomers on the basis of identical or different monomers of the polymeric chemistry. It is preferred to form the bridge composition by amino acids. The bridge composition must comprise a binding site for the solid phase. Usually, a positively charged binding site for the binding to a negatively charged solid phase, preferably a membrane, is provided.

Basically, the antigen/epitope sequences may be identical within a protein. It is particularly preferred, however, if the antigen/epitope sequences bind different antibodies. In an ideal manner, such different antigen/epitope sequences in a protein according to the invention or in a few proteins according to the invention are combined with each other, so that antibodies of the most usual (better all) sub-types of a species of causative agents are detected. The antigen/epitope sequences may for instance be repetitive sequence elements of identical or different HIV sub-types. It is preferred, however, if the antigen/epitope sequences are sequences of different HIV genes and/or strains and/or sub-types.

In principle, the bridge composition may be formed of any sequence, the bridge composition being for instance a sequence element of gp120. It is preferred if partial sequences unspecifically binding to antibodies contained in blood are deleted. This applies with regard to the bridge composition as well as to the antigen/epitope sequences.

The invention further relates to the application of proteins according to the invention for producing an immobilizate for the detection of antibodies, wherein first the protein is produced in a dissolved manner, then the protein being bound by at least one binding site to a solid phase, and as an option the solid phase with the protein bound thereto being subjected to at least one rinsing step and/or blocking step, and to the application of a protein according to the invention for performing a HIV test, an immobilizate according to the invention being produced and said immobilizate being placed in a housing, and a detector solution being brought-in in a reaction zone of the immobilizate or being separately added for application to the immobilizate.

The invention further relates to a polynucleotide, in particular cDNA, coding for a protein according to the invention, an expression vector, preferably plasmide, containing a polynucleotide sequence coding for a protein according to the invention, and a cell being transformed by means of an expression vector according to the invention.

Finally, the invention relates to a method for the production of a protein according to the invention, the antigen/epitope sequences and the bridge sequences being selected and the order of the lining-up being defined, wherein DNA coding for the antigen/epitope sequences and the bridge sequences is subsequently inserted into an expression vector in the defined manner and under application of a suitable promoter, a cell, preferably E. coli, being transformed by means of the expression vector and transformed cells being selected and cultivated, and wherein the protein expressed from the selected cells is isolated.

A HIV test according to the invention basically has the following structure. In a "flow through" version, a porous material, in most cases a membrane, is in a device, for instance a plastic housing having an access opening to the membrane. At surfaces of the membrane being accessible to liquids, at least one protein type according to the invention is immobilized. Through the access opening, a sample to be tested, for instance blood, serum or plasma, is applied on the membrane. Among other reasons, because of the capillary forces the sample will enter into the membrane or will pass through it. This can further be supported, for instance, by placing an absorbent material under the membrane. The sample or antibodies contained therein, respectively, react with the protein. By applying a conventional detector solution, for instance colored particles such as colloidal gold particles, a visual proof for the binding of antibodies to the protein is achieved. Alternatively to the "flow through" technology, a "lateral flow" method can be used. Therein a membrane is used, too, being however separated in a lateral direction into an application zone and a reaction zone. Caused by the capillary effect, the sample applied in the application zone will pass into the reaction zone where at least one protein type according to the invention is immobilized. In the application zone, there will be no proteins according to the invention immobilized, it is however possible to immobilize other substances, e.g. proteins, not binding the antibodies to be detected, however binding substances not desired in the sample and thus separating them. Thereby the reliability of the antibody detection is substantially improved, since undesired interactions because of other blood components can be excluded. The reaction zone may already contain a detector substance, however it may also possible that in the detector zone separately an application of detector solution is made.

The production of a total sequence of antigen/ epitope sequences and interposed bridge compositions being charged, for instance positively charged, has further special preparative advantages. If such a protein is produced gene-technologically by expression in a cell, first a purification of the protein from a cell extract is useful. The charges of the bridge compositions permit now a particularly effective and simple purification to very high purity levels in an ion exchange chromatography, since the isoelectrical point is extremely high. By such purification of a protein produced by gene technology according to the invention before the immobilization method step, thus a particularly pure immobilizate with regard to bound protein is obtained. This increases the reliability of a test system to a substantial degree.

### Definitions

The term HIV test designates a detection method for HIV antibodies in body components, in particular body liquids. Body liquids are for instance blood, serum, plasma, saliva, urine or liquor.

The term protein comprises, in the framework of the invention, compositions containing natural or non-natural amino acid sequences. A protein may be synthesized or at any case isolated with regard to the amino acid sequences. Insofar the term protein also comprises peptides. A protein needs not necessarily be formed of amino acids only. In particular the bridge composition may be structured differently than of amino acids.

As an antibody is designated a substance formed by an immune reaction in an organism in a natural manner after an infection, such substance being capable to specifically bind to the causative agent of the infection or components thereof.

As an antigen is designated a substance being capable to specifically bind to an antibody. Antigens and antibodies are assigned to each other on the basis of the antibody or antibodies to be detected.

An antigen/epitope sequence is an amino acid sequence, formed of natural and/or non-natural amino acids on one hand being chemically bindable to an assigned antibody, and on the other hand having such a secondary and/or tertiary structure that the purely chemical binding is also possible in a sterical manner, i.e. to the "key/lock principle".

The term immobilization designates the binding of a substance from the liquid phase to a solid phase surface. The term binding comprises the chemisorption, the ionic binding, the covalent binding and intermediate types of such bindings.

A bridge composition typically is an oligomer composition, with the kind of monomers, the order or sequence and the spatial orientation thereof including any internal or external linkage being defined. In particular are defined the positions of the ends of the bridge composition with respect to each other. It might happen that the antigen/epitope sequence respectively connected or to be connected and the opposing bridge composition are to be considered, if the number of the degrees of freedom of the bridge composition does not easily permit a sufficiently clear definition of the spatial position of the ends. A bridge composition is not necessarily an artificial sequence or a sequence artificially inserted between two antigen/epitope sequences. It may also be a native sequence between two naturally spaced antigen/epitope sequences, wherein one or more amino acids may also be exchanged.

A binding site is, in the terminology of the patent claims, a reactive group of the bridge composition, by means of which an immobilization at the solid phase can be achieved.

Exposure of an antigen/epitope sequence is the setting of the secondary and/or tertiary structure of the sequence such that a binding to the destination antibody can be performed. In particular this means the folding in the area of the antigen/epitope sequence. Folding may also take place with the integration of generating disulfide bridges. The folding will usually lead to the generation of a specific loop. An incorrect folding will lead to an incorrect loop. An incorrect folding may take place, if the ends of an antigen/epitope sequence are stabilized relative to each other in a not suitable manner.

With the feature of the bridge composition it is basically achieved that the parts of two bridge compositions being arranged between two adjacent binding sites will stabilize the ends of the interposed antigen/epitope sequence such that the desired folding is obtained. It is essential, herein, that the two binding sites are geometrical fixed points, due to the binding to the solid phase. Of course, non-functional sequences may be interposed between the end of a bridge composition and the antigen/epitope sequence connected thereto, as long as this will not affect the conditions described above.

A bridge sequence is a bridge composition being formed of multiple natural and/or non-natural amino acids.

The term of the different antibodies means antibodies of different type or of different structure. Corresponding considerations apply to genes, strains, sub-types and the like.

The term specificity designates the capability of a substance to detect out of a number of interaction possibilities or reaction possibilities provided a certain one or a group of certain ones. An antigen being specific for a certain antibody or a certain binding site of an antibody will have no reaction with other antibodies not having this binding site and being present in a sample, possibly after separation of the substances disturbing this binding site. In contrast thereto, antigens or sequences binding a multitude of different antibodies in a sample are not specific.

The term detection designates the generation of an arbitrary detector signal, observable directly by means of the human senses or indirectly by means of physical/chemical measuring methods, said detector signal having its cause in an antibody/antigen binding event. In the simplest case, it is a color reaction. Detection methods for antibody/antigen binding events are known in various ways to the man skilled in the art and need not be explained here in more detail.

A detector solution comprises one or more substances being capable to generate a detector signal upon an antibody/antigen binding event. The term solid phase designates a solid body with a surface capable to bind binding sites.

A rinsing step comprises rinsing of a generated immobilizate, i.e. a solid phase with a protein bound thereto, with a solution removing weakly bound proteins and/or other substances from the surface of the solid phase.

A blocking step comprises rinsing of an immobilizate with a solution comprising one or more substances, saturating those zones of the surface of the solid phase not gone in binding condition with the protein, i.e. blocking them for the binding of other substances directly at the surface of the solid phase.

### Embodiments of the invention

In the following, not limiting examples only of embodiments of the invention are described in more detail.

### Example 1.

In the following, examples for artificial bridge compositions are given which can be used for a protein according to the invention for the detection of HIV antibodies.

The bridge composition 1 may comprise the following sequence: GKR--K-RK-KR--RRG with "-" representing one or more of any amino acids. An example for such a bridge composition is:

GKRAHKSRKHNYKRHIRRG

The bridge composition 2 may comprise the following sequence: G-KK-RR-KGK-RR-KK-G with "-" representing one or more of any amino acids. An example for such a bridge composition is:

GSKKARRIKGKMRRLKKVG

The bridge composition 3 may comprise the following sequence: G-C-K-R-KRKXKRK-K--C-G with "-" representing one or more of any amino acids, and "X" representing D or E. An example for such a bridge composition is:

GVCIKHRYKRKDKRKHKVACIG

The letters in the sequence information above and below are based on the single letter code.

### Example 2.

In the following, a native bridge composition is stated which is suitable for a protein according to the invention for the detection of HIV antibodies. GVA--K-KRR---REKRAVG with "-" representing an arbitrary amino acid. This bridge composition comes from the HVI-1 envelope gene.

### Example 3.

In the following, proteins according to the invention under application of bridge compositions, e.g. from examples 1 and/or 2, are described in more detail. Table 1 supplies possibilities of the deletion and/or insertion of bridge compositions in sequences with antigen/epitopes. Suitable sequences ID I to ID VI with antigen/epitopes are shown in Figure 1a-f, these being complete sequences of HIV proteins (for the purpose of the present invention, it is however not necessary to use complete sequences).

Table 1 is to be read in such a way that in the leftmost and the leftmost but one column a starting protein, ID I to ID VI, being suitable for the generation of a protein according to the invention is shown. The further columns indicate whether and where deletions and/or insertions with bridge compositions, for instance according to table 2, can be inserted into the starting protein in order to obtain a protein according to the invention. In table 2 are: ID 2 to 3 artificial bridge compositions, ID 4 a natural bridge composition (from the transition gp120/gp41 in env), ID 5 to 8 HIV-1 0-sequences, ID 9 to 12 Gnann region from HIV-2 and ID 13 to 36 V3 loop sequences from HIV-1 (consensus sequences of the respective sub-types and sequences similar thereto, also from sub-type 0).

A protein according to the invention can for instance be obtained, if in pol 2 having 289 amino acids at position 31 either the bridge composition 1 or the bridge composition 2 from table 2 are inserted, not however bridge composition 3. As a result, the unlimited combination possibilities stated in table 1 are obtained.

From the graphic overviews of figures 1a-f, deletions and possible insertion positions of table 2 can also be taken. In the sequences, the symbol "+1/2/3+" corresponds to the insertion of the bridge composition 1, 2 or 3. "+1/2+" also stands for an insertion of one of the bridge compositions 1 or 2.

### Example 4.

In Fig. 2 are shown the sequences Seq. ID A to E, with A to D being proteins according to the invention, E however a protein not according to the invention. In the sequences A to D modifications are performed such that between the antigen/epitope sequences suitable bridge compositions will be formed. In contrast thereto, in E will not take place a sufficient presentation of antigen/epitope sequences. In Fig. 3a/b, the structures of the proteins C to E are shown, and reference is made to the following examples.

### Example 5.

In this example, the production of a protein according to the invention is described, namely of p31 Δ100/40,140/23,163/14 Ω31/2,100/3
with the meaning:
ΔX/Y (e.g. Δ100/76): behind the amino acid at position 100, 76 amino acids are deleted, and
ΩX/Y (e.g. Ω30/2) : behind the amino acid at position 30, bridge composition 2 is inserted.

### Partial step 1

The fragment 1 is obtained by the PCR technology. As primers for the amplification are used the oligonucleotides 5′-ATATGGCATATGTTTTTAGATGGAATAGATAAGG CCC-3′ and 5'-TATAGGGCCCAGGTGGCAGGTTAAAA-3' as a template the HIV-pol gene is used. The PCR reaction is performed to a conventional protocol. The desired PCR product (approx. 110 bp) is isolated and subjected to a restriction with ApaI and NdeI.

Fragment 1 (epitope 1, PCR)

NdeI
atatggCATATGTTTTTAGATGGAATAGATAAGGCCC
........TTTTTAGATGGAATAGATAAGGCCCAAGATGAACATGAGAAATATCACAGTAATTGGAGA
M F L D G I D K A Q D E H E K Y H S N W R
........AAAAATCTACCTTATCTATTCCGGGTTCTACTTGTACTCTTTATAGTGTCATTAACCTCT

GCAATGGCTAGTGATTTTAACCTGCCACCT..................
A M A S D F N L P P
CGTTACCGATCACTAAAATTGGACGGTGGA..................
AAAATTGGACGGTGGACCCGGGatat
ApaI

### Partial step 2

Two oligonucleotides 5'-CAAAAAGGCCCGTCGCATCAAGGGCAAAATGCGACGGGTGAAGAAAG-3' and 5′-CCGGCTTTCTTCACCCGTCGCATTTTGCCCTTGATGCGACGGGCCTTTTTGGGCC-3′ are denaturated for a short time at 100 °C, hybridized with each other at a linearly dropping temperature gradient, and the double-strand product is then isolated.

Fragment 2 (bridge composition 2, synthetic)

ApaI
CAAAAAGGCCCGTCGCATCAAGGGCAAAATGCGACGGGTGAAGAAAG
G P K K A R R I K G K M R R V K K A G
CCGGGTTTTTCCGGGCAGCGTAGTTCCCGTTTTACGCTGCCCACTTCTTTCGGCC
NgoMIV

### Partial step 3

The fragment 3 is obtained by PCR technology. As primers for the amplification are used the oligonucleotides 5′-TAATTTGCCGGCGTAGTAGCAAAAGAAATAGTAG-3′ and 5′-TATAGCATGCTCCATATGCTGTTTCCTGCCCTGT-3′ as a template the HIV-pol gene is used. The PCR reaction is performed to a conventional protocol. The desired PCR product (233 bp) is subjected to a restriction with NgoMIV and SphI and is then isolated.

Fragment 3 (epitope 2, PCR) (233 bp)

NgoMIV
taatttGCCGGCGTAGTAGCAAAAGAAATAGTAG
GTAGTAGCAAAAGAAATAGTAGCCAGCTGTGATAAATGTCAGCTAAAAGGAGAAGCCATG
A G V V A K E I V A S C D K C Q L K G E A M

CATGGACAAGTAGACTGTAGTCCAGGAATATGGCAACTAGATTGTACACATTTAGAAGGA
H G Q V D C S P G I W Q L D C T H L E G

AAAGTTATCCTGGTAGCAGTTCATGTAGCCAGTGGATATATAGAAGCAGAAGTTATTCCA
K V I L V A V H V A S G Y I E A E V I P

GCAGAAACAGGGCAGGAAACAGCATAT
A E T G Q E T A Y G A C
CGTCTTTGTCCCGTCCTTTGTCGTATA
TGTCCCGTCCTTTGTCGTATACCTCGTACGatat
SphI

### Partial step 4

Two oligonucleotides 5′-CATGCATCAAACACCGCTACAAGCGACGCGATCGTCGGAAGCATAAAGTGGCCTGC- 3′ and 5'-CTAGGCAGGCCACTTTATGCTTCCGACGATCGCGTCGCTTGTAGCGGTGTTTGATG-3' are denaturated for a short time at 100 °C, hybridized with each other at a linearly dropping temperature gradient, and the double-strand product is then isolated.

Fragment 4 (bridge composition 3, synthetic)

SphI
CATGCATCAAACACCGCTACAAGCGACGCGATCGTCGGAAGCATAAAGTGGCCTGC
A C I K H R Y K R R D R R K H K V A C I G
GTAGTTTGTGGCGATGTTCGCTGCGCTAGCAGCCTTCGTATTTCACCGGACGGATC
AvrII

### Partial step 5

The fragment 5 is obtained by PCR technology. As primers for the amplification are used the oligonucleotides 5'-ATTATCCTAGGTCAAATGGCAGTATTCATCCAC-3' and 5'-TATAGGATCCTAATCCTCATCCTGTCTACTTGC-3' as a template the HIV-pol gene is used. The PCR reaction is performed to a conventional protocol. The desired PCR product (360 bp) is subjected to a restriction with AvrII and BamHI and is then isolated.

Fragment 5 (epitope 3, PCR)

AvrII
attatCCTAGGTCAAATGGCAGTATTCATCCAC
CAAATGGCAGTATTCATCCACAATTTTAAAAGAAAAGGGGGGATTGGGGGGTACAGTGCA
C I G Q M A V F I H N F K R K G G I G G Y S A
GGGGAAAGAATAGTAGACATAATAGCAACAGACATACAAACTAAAGAATTACAAAAACAA
G E R I V D I I A T D I Q T K E L Q K Q
ATTACAAAAATTCAAAATTTTCGGGTTTATTACAGGGACAGCAGAAATCCACTTTGGAAA
I T K I Q N F R V Y Y R D S R N P L W K
GGACCAGCAAAGCTCCTCTGGAAAGGTGAAGGGGCAGTAGTAATACAAGATAATAGTGAC
G P A K L L W K G E G A V V I Q D N S D
ATAAAAGTAGTGCCAAGAAGAAAAGCAAAGATCATTAGGGATTATGGAAAACAGATGGCA
I K V V P R R K A K I I R D Y G K Q M A
GGTGATGATTGTGTGGCAAGTAGACAGGATGAGGATTAG
G D D C V A S R Q D E D *
CCACTACTAACACACCGTTCATCTGTCCTACTCCTAATC
CGTTCATCTGTCCTACTCCTAATCCTAGGatat
BamHI

### Partial step 6

A suitable expression vector is cut with NdeI and BamHI, and the vector fragment is isolated.

### Partial step 7

The purified vector fragment and the respectively isolated partial fragments are linked to each other by means of ligation reactions.

### Partial step 8

Suitable E. coli cells are transformed and selected with the ligation products.

### Partial step 9

From obtained colonies is isolated the plasmide DNA and tested for the presence, arrangement and orientation of the partial sequences with the enzymes NdeI, ApaI, NgoMIV, SphI, AvrII and BamHI in different combinations. Plasmide DNA with a positive result is sequenced for confirmation.

### P31 native

P31Δ100/40,140/23,163/14 Ω31/2,100/3
Frg.1 Frg.2 Frg.3 Frg.4 Frg.5

NdeI ApaI NgoMIV SphI AvrII BamHI

Total nucleotide sequence:

CATATGTTTTAGATGGAATAGATAAGGCCCAAGATGAACATGAGAAATATCACAGTA
ATTGGAGAGCAATGGCTAGTGATTTTAACCTGCCACCTGGGCCCAAAAGGCCCGTCG
CATCAAGGGCAAAATGCGACGGGTGAAGAAAGCCGGCGTAGTAGCAAAAGAAATAGTA
GCCAGCTGTGATAAATGTCAGCTAAAAGGAGAAGCCATGCATGGACAAGTAGACTGTA
GTCCAGGAATATGGCAACTAGATTGTACACATTTAGAAGGAAAAGTTATCCTGGTAGC
AGTTCATGTAGCCAGTGGATATATAGAAGCAGAAGTTATTCCAGCAGAAACAGGGCAG
GAAACAGCATATGGAGCATGCATCAAACACCGCTACAAGCGACGCGATCGTCGGAAGC
ATAAAGTGGCCTGCCTAGGTCAAATGGCAGTATTCATCCACAATTTTAAAAGAAAAGG
GGGGATTGGGGGGTACAGTGCAGGGGAAAGAATAGTAGACATAATAGCAACAGACATA
CAAACTAAAGAATTACAAAAACAAATTACAAAAATTCAAAATTTTCGGGTTTATTACA
GGGACAGCAGAAATCCACTTTGGAAAGGACCAGCAAAGCTCCTCTGGAAAGGTGAAGG
GGCAGTAGTAATACAAGATAATAGTGACATAAAAGTAGTGCCAAGAAGAAAAGCAAAG
ATCATTAGGGATTATGGAAAACAGATGGCAGGTGATGATTGTGTGGCAAGTAGACAGG
ATGAGGATTAGGATCC

Total amino acid sequence:

MFLDGIDNAQDEHEKYHSNWRAMASDFNLPPGPKKARRIKGFMRRVKKAGVVAKEIVA
SCDKCQLKGEAMHGQVDCSPGIWQLDCTHLEGKVILVAVHVASGYIEAEVIPAETGQE
TAYGACIKHRYKRRDFRKHKVACIGQMAVFIHNFKRKGGIGGYSAGERIVDIIATDIQ
TKELQKQITKIQNFRVYYRDSRNPLWKGPAKLLWKGEGAVVIQDNSDIKVVPRRKAKI
IRDYGKQMAGDDCVASRQDED*

The expression of a protein according to the invention is performed as follows. After the coding nucleic acid for the expressing protein having been produced and built-in over restriction interfaces into a commercially available vector, a transformation of the plasmide into a commercially available E. coli cell takes place. After inoculation of a medium (e.g. LB medium) with the transformation addition, the breeding of the cell culture by incubation is performed at an optimum temperature (e.g. 37 °C). The protein expression is induced by addition of isopropyl β-D thiogalactopyranoside (IPTG) and achieved by continuation of the incubation. After harvesting the E. coli cells by centrifugation, a cell lysis with a lysis buffer (for instance a guanidine hydrochloride containing buffer).

Purification of the protein can be achieved by conventional chromatography methods (for instance ion exchange chromatography and gel chromatography), the isoelectrical point of the proteins increased by the insertion of positively charged sequences being used in an advantageous manner to substantially enrich the proteins by cation exchange chromatography. Usually the chromatographic separations are performed by means of chromatography columns brought into the material. If the proteins have been provided with a "tag" (e.g. a N or C-terminal His6 peptide, a "flag tag" or a myc epitope), they can be purified by utilization of an affinity chromatography, the respective protein being bound by the "tag" to the corresponding affinity chromatography material (e.g. for the His6 tag to Ni-NTA material). After the material having been washed several times with corresponding washing buffers, the desired protein is separated from the material by means of at least one elution buffer (for instance by a buffer with substantially higher or lower pH value). Finally, the eluates are subjected to several dialysis steps, in order to permit further use of the protein.

### Example 6.

In this example, the production of an immobilizate according to the invention is described. The purified protein is diluted in a suitable manner with an aqueous solution of 100 mM NaCl and 0.4 - 0.8 % SDS. From this solution, 0.5 µl is applied by means of a BioDot device (Cambridge, UK) on a nitrocellulose membrane, so that on a spot of 3 - 4 mm diameter there are 250 ng protein. After application of the protein, the membrane is dried for at least one hour, before the detection reaction is performed.

An immobilizate is obtained, wherein the arrangement and the folding of the protein is achieved in a way that is diagrammatically shown in Fig. 3a. The epitopes are separated by a positively charged peptide sequence, permitting an adhesion to the membrane and a correct folding of the epitopes (underlined sequence sections).

It may be noted, with regard to the folding shown in Fig. 3a, that possibly not all protein molecules applied on the solid phase can be folded in the sketched way. Part of the molecules may, caused by the lysis of the cells and the subsequent purification procedure, be present in a disordered (denaturated) folding condition. For the purpose of the present invention it is however sufficient, if for a protein structured according to the invention the probability of a folding suitable for the binding of antibodies, induced by the optimum adhesion on the solid phase permitted by the integration of the bridge compositions, is clearly increased in comparison to a protein containing no bridge compositions. By this increased probability of the "correct" folding , the possibility of the binding of antibodies is in total substantially improved, and thus the increase in sensitivity of the whole detection method is secured.

### Example 7.

To an immobilizate from example 6 have been fed serums of various HIV positive patients, and the reaction was examined by means of a detector solution. In all cases a color reaction specific for the binding antibody/antigen was observed. Incorrect negative results were not obtained. Tests with serums of HIV negative patients showed that not a single incorrect positive result was obtained.

### Example 8.

In this comparison example, an immobilizate has been produced with a protein env 4 (ID E) according to the procedure of example 6. The difference is however, as can be seen from a comparison of figures 3a and 3c, that between the epitopes arranged at the left side there is no positively charged bridge composition. It can further be seen that thereby the two epitopes on the left side are closely arranged to each other and are folded - under formation of disulfide bridges destroying the antigenicity - such that binding of an antibody cannot be achieved.

With the same protein amount as in example 7, again tests with serums of various HIV positive patients have been made. Without exception, no reaction was shown, that is regularly incorrect negative results were obtained.

### Example 9.

In this example a protein (ID D) according to the invention or an immobilizate with repetitive sequence elements of different sub-types (identical ones are also possible) is diagrammatically represented with reference to Fig. 3b. This protein according to the invention is formed of V3 loops only of different HIV sub-types being separated by a positively charged sequence element of gp120.

### Example 10.

A rapid test according to the invention is configured as follows. An immobilizate according to example 6 is positioned and fixed in a plastic housing underneath an access opening such that at least part of the immobilizate is open for access. The immobilizate is placed on an absorbing material, for instance cotton wool. A separately packed detector solution belongs to the rapid test.

The detection of antibodies bound to the antigen may be performed by means of a detection solution containing a conjugate of protein A with colloidal gold. The application of such conjugates in immunological test methods is generally known in the art and has been described in the literature. This conjugate may for instance be produced, as described in US 5,541,059, by mixing 100 ml of a colloidal gold solution (commercially available) with 100 ml of 0.006 mg/ml protein A solution (protein A is commercially available in a lyophilized form and as a solution). Alternatively a protein A-gold conjugate may commercially be acquired.

The binding of the conjugate to the bound antibodies can visually be detected. In this example, a red stain will appear if bound antibodies are present.

Alternatively to the conjugates with colloidal gold a dye suspension can be used, with protein A being adsorbed to a water-insoluble dye.

A rapid test is performed as follows. A human guinea pig is lightly injured by a prick. The resulting drop of blood is received in a small capillary. The capillary with the blood is then given into a container together with a suitable solvent, for instance physiological sodium chloride solution, and is agitated until blood and solvent are fully mixed. The contents of the container is then fed through the access opening on the immobilizate. Then, the detector solution is added, and it is visually monitored whether a coloration of the immobilizate visible through the access opening takes place. Coloration means that HIV antibodies exist in the blood of the human guinea pig. If there is no coloration, the result is negative.

## Claims

1. A protein having multiple antigen/epitope sequences for antibodies, wherein the protein is immobilized at a solid phase by at least one binding site, and the antigen/epitope sequences are spaced by bridge compositions in such a way that after binding of the binding site at the solid phase the antigen/epitope sequences are exposed for a binding of the assigned antibodies from the liquid phase.

2. A protein according to claim 1, wherein a bridge composition is formed by insertion of bridge sequences between two antigen/epitope sequences and/or deletion of a partial sequence between two antigen/epitope sequences arranged in a total sequence.

3. A protein according to claim 1, wherein the bridge composition is formed by fusion of a bridge sequence with two antigen/epitope sequences.

4. A protein according to one of the claims 1 to 3, wherein the bridge composition comprises positively charged binding sites for the binding to a negatively charged solid phase, preferably a membrane.

5. A protein according to one of claims 1 to 4, wherein the antigen/epitope sequences bind different antibodies.

6. A protein according to one of claims 1 to 5, wherein the antigen/epitope sequences are repetitive sequence elements of identical or different HIV sub-types.

7. A protein according to one of claims 1 to 5, wherein the antigen/epitope sequences are sequences of different HIV genes and/or strains and/or sub-types.

8. A protein according to one of claims 1 to 5, wherein the antigen/epitope sequences are sequences of a single HIV sub-type.

9. A protein according to one of claims 1 to 8, wherein the bridge composition is a sequence element of gp120.

10. A protein according to one of claims 1 to 9, wherein partial sequences unspecifically binding to antibodies contained in blood are deleted.

11. The application of a protein according to one of claims 1 to 10 for the production of an immobilizate for the detection of antibodies, wherein first the protein is produced in a dissolved manner, then the protein is bound by at least one binding site to a solid phase, and as an option the solid phase with the protein bound thereto is subjected to at least one rinsing step and/or blocking step.

12. The application of a protein according to one of claims 1 to 10 for performing a HIV test, wherein an immobilizate according to claim 11 is produced and said immobilizate is placed in a housing, and wherein a detector solution is brought-in in a reaction zone of the immobilizate or is separately added for application to the immobilizate.

13. A polynucleotide, in particular cDNA, coding for a protein according to one of claims 1 to 10.

14. An expression vector, preferably plasmide, containing a polynucleotide sequence coding for a protein according to one of claims 1 to 10.

15. A cell which is transformed by means of an expression vector according claim 14.

16. A method for the production of a protein according to one of claims 1 to 10, wherein the antigen/epitope sequences and the bridge sequences are selected and the order of the lining-up is defined and DNA coding for the antigen/epitope sequences and for the bridge sequences is subsequently inserted into an expression vector in the defined manner, a cell, preferably E. coli, being transformed by means of the expression vector and transformed cells being selected and cultivated, and wherein the protein expressed from the selected cells is isolated.
